# EUROPEAN PATENT SPECIFICATION

(11) **EP 1 599 134 B1**
(45) Date of publication and mention of the grant of the patent: **01.10.2014**
(21) Application number: 03783771.3
(22) Date of filing: 25.11.2003
(51) Int. Cl.: A61B 5/05, A61B 18/00, A61B 17/34, A61F 7/12

(54) **SYSTEM FOR PROVIDING COMPUTER GUIDED ABLATION OF TISSUE**
SYSTEM ZUM BEREITSTELLEN VON COMPUTERUNTERSTÜTZTER GEWEBEENTFERNUNG
SYSTEME PERMETTANT D'EFFECTUER UNE ABLATION DE TISSU GUIDEE PAR ORDINATEUR

(30) Priority: 27.11.2002 US 307036; 03.11.2003 US 700326
(43) Date of publication of application: 30.11.2005
(73) Proprietor: Endocare, Inc., Austin, TX 78717 (US)
(72) Inventor: DAMASCO, Sanford, D., Irvine, CA 92620 (US); ALI, Jawahar, M., Lake Forest, CA 92630 (US); BATTLES, David, J., Haleiwa, HI 96712 (US); MIKUS, Paul, W., Irvine, CA 92620 (US); EUM, Jay, J., Irvine, CA 92602 (US); DUONG, Thach, Tustin, CA 92782 (US)
(74) Representative: Emde, Eric
(86) International application number: PCT/US2003/037643
(87) International publication number: WO 2004/051409

(56) References cited:
- WO-A-98/23214
- US-A- 5 897 495
- US-A- 6 095 975
- US-A1- 2002 016 540
- US-A1- 2002 022 869
- US-B1- 6 241 725
- US-B1- 6 311 084
- US-B2- 6 539 247

## Description

### BACKGROUND OF THE INVENTION

The present invention relates to cancer surgery and more particularly to a computer guided system for ablative surgery with enhanced feedback.

There is reference in the prior art to the use of computer control systems for providing and/or enhancing cryosurgical techniques. For example, U.S. Pat. No. 4,672,963, issued to I. Barken, discloses an automated and integrated system including a cryosurgery device, an imaging probe and a computer system for use in performing internal surgery.

U.S. Pat. No. 5,647,868, issued to D.O. Chinn, discloses another cryosurgical integrated control and monitoring system.

U.S. Pat. No. 6,139, 544, issued to P.W. Mikus et al, discloses a system for assisting surgeons in performing cryosurgery of the prostate by calculating optimal positions for cryoprobes and providing display based templates for overlay over an ultrasound image display, and displaying actual cryoprobe ultrasound images together with template images so that the surgeon may compare suggested and actual placement of the cryoprobes, and adjust placement accordingly.

The presently utilized CryoCare™ Surgical System which is currently manufactured and marketed by Endocare, Inc., Irvine, CA. utilizes cryoprobes to deliver cold temperatures to the targeted tissue and temperature probes (marketed under the trademark TempProbe®) to monitor temperatures in the surrounding tissue. The CryoCare™ Surgical System presently requires a certain degree of skill for operation since the physician requires an understanding of the temperature mapping of the cryoprobes in order to operate them to deliver an effective treatment.

U.S. Pat. No. 6,241,725 relates to the destruction of pathological volumes or target structures such as cancerous tumors or aberrant functional target tissue volumes by direct thermal destruction. In the case of a tumor, the destruction is implemented in one embodiment of the invention by percutaneous insertion of one or more radiofrequency probes into the tumor and raising the temperature of the tumor volume by connection of these probes to a radiofrequency generator outside the body so that the isotherm of tissue destruction enshrouds the tumor. The ablation isotherm may be predetermined and graded by proper choice of electrode geometry and radiofrequency (rf) power applied to the electrode with or without temperature monitoring of the ablation process. Preplanning of the rf electrode insertion can be done by imaging of the tumor by various imaging modalities and selecting the appropriate electrode tip size and temperature to satisfactorily destroy the tumor volume. Computation of the correct three-dimensional position of the electrode may be done as part of the method, and the planning and control of the process may be done using graphic displays of the imaging data and the rf ablation parameters. Specific electrode geometries with adjustable tip lengths are included in the invention to optimize the electrodes to the predetermined image tumor size.

The computer workstation suggested in US 6,241,725 only functions as a visualization system for real-time monitoring of the lesion process during the ablation operation, and it can be concluded that the pre-planning of US 6,241,725 is meant to process the output data in light of a database or mode known and show the status of operation.

Document WO 98/23214 discloses a system for providing computer guided ablation of tissue according to the preamble of claim 1.

In accordance with the present invention, a system for providing computer guided ablation of tissue of a patient as set forth in claim 1 is provided. Further embodiments of the invention are disclosed in the dependent claims.

### SUMMARY OF THE INVENTION

The present invention is a system for providing computer guided ablation of tissue of a patient according to claim 1.

The feedback described in claim 1 provides enhanced automation and minimizes the potential for operator error resulting in an ineffective or unsuccessful treatment. This enhancement to the Cryocare™ Surgical System, discussed above, will be marketed by the present assignee, Endocare, Inc., under the trademark AutoFreeze™.

### BRIEF DESCRIPTION OF THE DRAWINGS

Figure 1 is an overall system schematic of the present invention.
Figure 2 is a schematic perspective view, partially in cross section of the components of the system for providing computer guided ablation, of the present invention.
Figure 3 is a sample display screen of the computer system of the present invention.
Figure 4 is a flow diagram of the treatment module of the present invention.
Figure 5 is an illustration of the prostate showing cryoprobe and temperature probe placement.
Figure 6 is a flow diagram of the overall ablation cycle of the present invention.
Figure 7a is flow diagram of the freeze cycle for the first anterior cryoprobe and the second anterior cryoprobe.
Figure 7b is a flow diagram of the freeze cycle for the first posterior lateral cryoprobe and the second posterior lateral cryoprobe.
Figure 8 is a front view of the alignment assembly showing the cryoprobes and temperature probes being placed at selected locations.

### DETAILED DESCRIPTION OF THE INVENTION

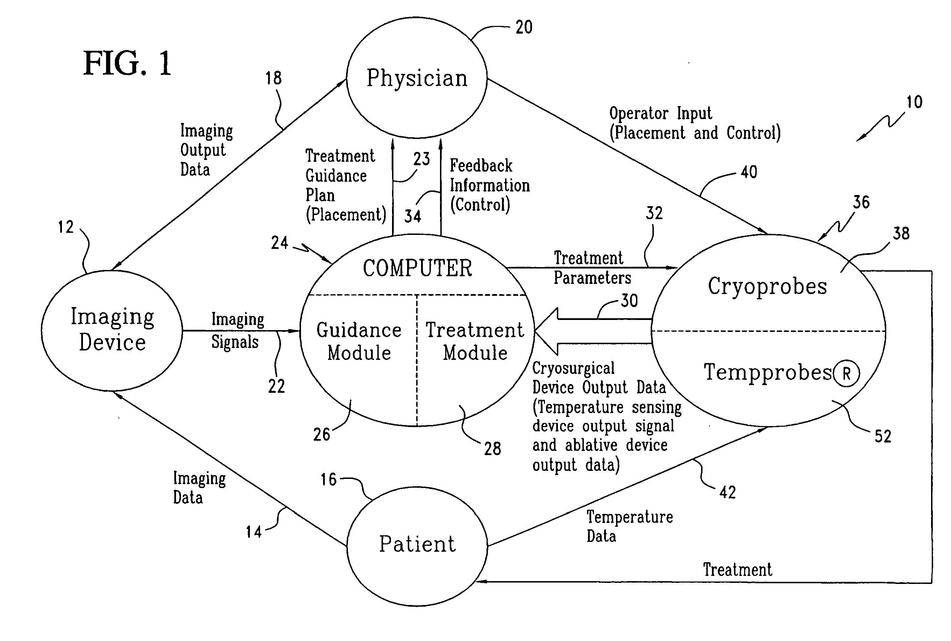

Referring now to the drawings and the characters of reference marked thereon, Figure 1 illustrates a preferred embodiment of system for providing computer guided ablation of tissue, of the present invention, designated generally as 10. The system 10 includes an imaging device 12, such as ultrasound, MRI, CT, PET, SPECT, X-ray (including fluoroscope) or other suitable imaging device. The imaging device 12 receives imaging data 14 from a treatment region of a patient 16. The treatment region may be, for example, the prostate region, breast region, liver region, etc. The imaging device 12 provides imaging output data 18 to the physician or other operator 20 and imaging signals 22 to an ablative surgical computer system, designated generally as 24.

The ablative surgical computer system 24 includes a guidance module 26 for processing the imaging signals 22 and providing a treatment guidance plan 23 to the operator 20. The computer system 24 also includes a treatment module 28 for acquiring and processing surgical device output data 30, for optimally controlling treatment parameters 32 and providing feedback information 34 to the operator 20 based on the treatment guidance plan 23.

A set of surgical devices, designated generally as 36, includes at least one ablative device 38 for providing ablation of the treatment region based on the treatment parameters 32 and operator input 40. The set 36 of surgical devices also includes at least one temperature sensing device 52 for acquiring temperature data 42 from the treatment region of the patient 16. The set 36 of surgical devices provides the surgical device output data 30. A temperature sensing device output signal is provided which is a portion of the surgical device output data 30.

In the present invention the ablative devices 38 are cryosurgical probes, as will be explained in detail below. However, it is understood that various other types of ablative devices 38 may be used to provide the necessary ablation. The ablative devices 38 may comprise, for example, radio frequency electrodes, laser fibers, microwave catheters, high-intensity focused ultrasound, and other suitable ablative devices.

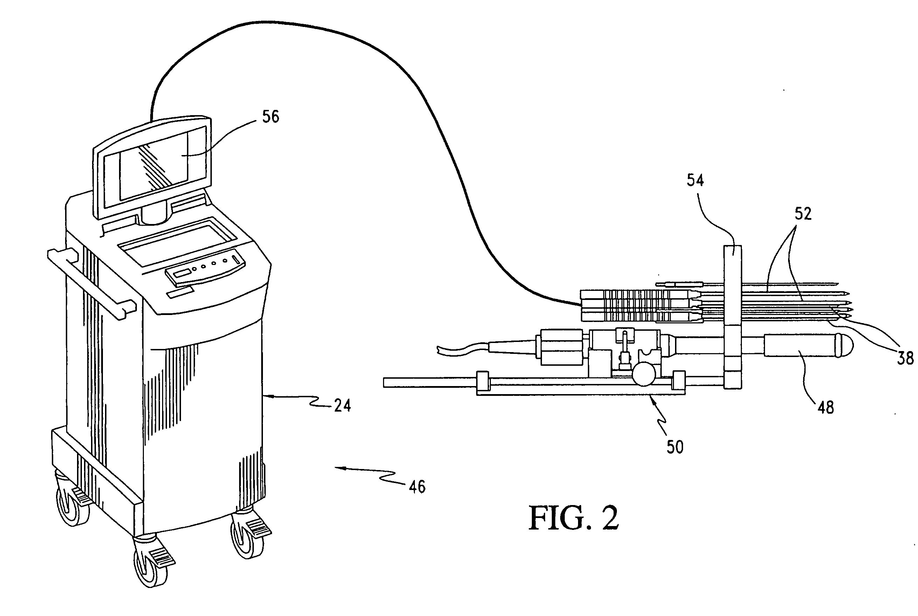

Referring now to Figure 2 utilization of the present system with cryosurgical probes (38), which function to ablate tissue, is illustrated, designated generally as 46. The surgical computer system 24, in present applicants' present application provides guidance as to recommended ablative element placement within a prostate 13, based on images of the prostate acquired from the imaging system, such as an ultrasound system, designated generally as 48.

The computer system 24 is programmed with software capable of: determining the dimensions of the prostate; determining the dimensions of a treatment zone; and, utilizing the determined dimensions of the prostate and treatment zone for computing the number and location of ablative elements needed to treat the treatment zone. An IBM-compatible microprocessor serves as the host computer.

The transrectal ultrasound probe 48 is used to visualize the prostate and the cryosurgical probes. A stepper assembly 50 provides the required advance. The ablative devices (e.g. cryoprobes 38) are illustrated as well as temperature probes 52. The set of surgical devices, i.e ablative devices and temperature sensing devices, are introduced through a grid (i.e. reference plate) 54.

Treatment planning preferably includes the following steps:

### Step 1

### Capturing Image

- The live ultrasound image is displayed in the ultrasound image window.
- A button entitled CAPTURE will appear at the bottom of the display.
- A brachy-type grid, i.e. grid having an orthogonal reference system, should be displayed on the ultrasound image before the first image is captured.
- Using the Capture window, click CAPTURE to capture the first image at the widest cross section of the prostate. Once CAPTURE is selected, the image will be frozen and displayed as a thumbnail image on the right-hand side of the screen.
- You can now remove the brachy grid display for the remaining captures.
At least one image is captured. However, there is an option, for example, to capture two images at the widest portion of the prostate gland, one with the brachy grid displayed and one without it displayed, then capture additional images at the base and apex of the gland.

### Step 2

### Calibration

Typically, there is a calibration step.

### Step 3

### Outlining

- You are asked to click on the four outer points of the prostate image displayed.
- Start by clicking on the top edge of the prostate.
- Next click on the outer most right hand side of the prostate.
- Repeat this action on the bottom edge and left hand outer edge of the prostate as directed in the step 3 window text and illustrations.
- When you have clicked on all four points, click the right mouse button to complete the outline.
- At anytime during the outlining process, the UNDO button in the step 3 window can be selected to remove the last point placed.
- When the prostate outline is completed, the system will move to the URETHRA contour mode.
- To outline the urethra click on the center of the urethra and a circle will be placed.
- You can adjust the urethra contour location by clicking in the center of the circle and dragging the circle to a new location holding the mouse button down.
- You can adjust the size of the urethra outline by clicking on one of the four white dots displayed outside of the outline and moving it inward to reduce the size or pulling it outward to increase the size.
- You must click the right mouse button to complete the urethral outline.
- When the urethra outline is completed, the system will move to the RECTAL WALL contour mode.
- To outline the rectal wall, click on the left top edge of the rectal wall and then click on the right top edge of the rectal wall.
- You can adjust the rectal wall outline by clicking on any of the points in the outline and dragging them to a different location.
- When the rectal wall outline is complete, right click to move to the next step.
Three image option:
- When all outlines on the first image are complete, the system will ask you to outline the urethra on the base and apex images.
- Outline the urethra in each additional image using the same method described previously.
- Right click each time an outline has been completed.

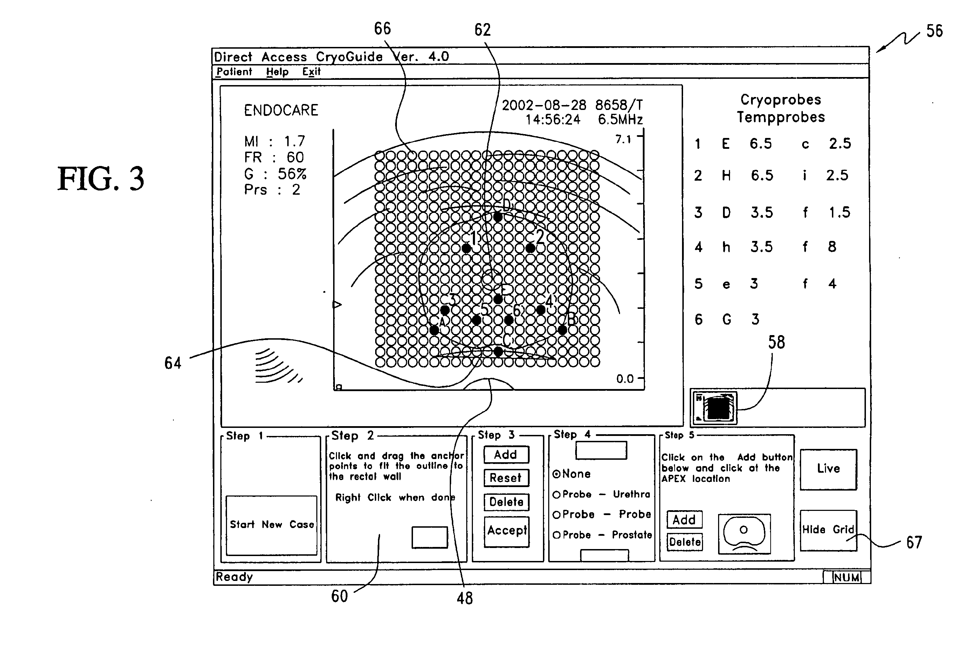

Referring now to Figure 3, a sample display screen, designated generally as 56, of the computer system 24 showing treatment planning is illustrated. The display screen 56 contains various sections. For example, a thumbnail section 58 displays thumbnail images.

Another section on the display screen 48 is the instruction box 60 that provides the user with detailed instructions at each step and makes the system easier to use. Additionally, the system has controls for specifying the patient details (name, age, etc.), calibration, adding/deleting probes and for the simulation of the ablation. The system also provides a pull down menu for switching rendering views and to toggle the display of the probe placements.

### Step 4

### Placing Probes

- The step 4 window and suggested probe placement will appear next to the step 3 window when the outlining is complete. This window allows you to move, add or delete probes if desired.
- Probe grid coordinates will also be displayed on the far right hand side of the screen.
- To move a probe from the suggested probe placement, click and drag the probe points displayed on the image. This will result in the probe coordinates changing to the new location.
- To add or delete probes, click the add or delete button and then click on the location on the image where you want to add a probe or click on the probe you want to delete. This will add or remove the probe to the coordinate display on the right hand side of the screen.
- Once the probes are in the desired locations, click on the accept button to proceed to step 6.

### Step 5

### Measure

This enables the user to display key distance measurements as well as view customized measurement distances.

### Step 6

### TempProbe® Temperature Probe Placement

- Step 6 allows the user to place TempProbes® in the desired location on the image and displays the grid coordinate points that correspond to that placement.
- The user is prompted to click on the locations for the right neurovascular bundle (RNVB), left neurovascular bundle (LNVB), Apex and External Sphincter (ES) TempProbes® in the image.
- For each placement the user must click on the add button in the step 6 window and then click on the location for placement in the image.
- A minimum of four TempProbes® should be placed.
- TempProbe® grid coordinates are displayed on the right hand side of the screen next to Cryoprobe coordinates.
- The user can click on the LIVE button in the bottom right hand corner of the screen to overlay the probe placement locations and grid on top of the live ultrasound image.
- The user can click on the same button that is now labeled captured images to return to the captured image display.
- The user can click on the Hide Grid/Display Grid button in the bottom right hand corner of the screen to toggle the Cryogrid overlay on and off.

Although the aforementioned treatment planning and placement steps have been described with reference to a drag ball or mouse interface device, it is understood that other interface devices can be used such as touch screens, joysticks, etc.

The ultrasound probe image 48 can be seen in Figure 3. Furthermore, parts of the anatomy can be seen, such as the urethra 62 and the rectum 64. The TempProbes® are denoted A, B, C, D and E. The cryoprobes are denoted by numeral designations 1-6. The grid being used is also shown in this display, as denoted by numeral designation 66. As noted above, the grid 66 can, optionally be deleted from the display by selecting the "hide grid" option 67.

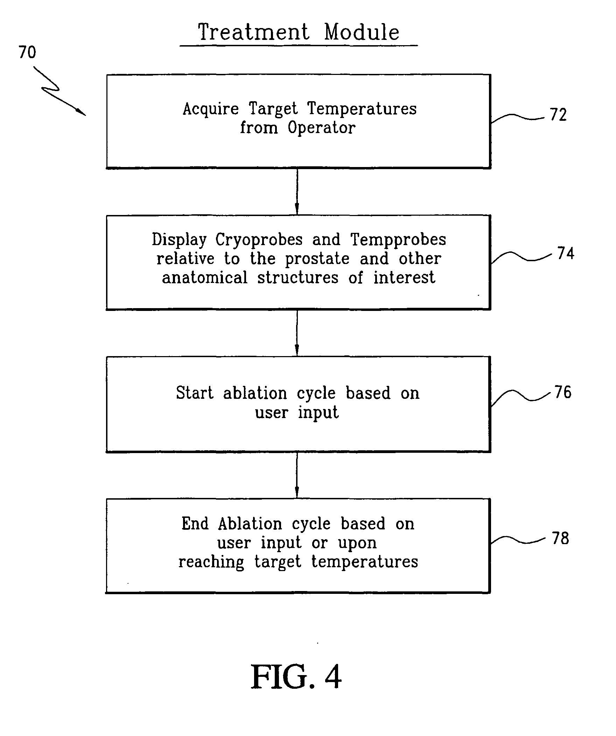

Referring now to Figure 4, a flow diagram for the treatment module 70, is illustrated. Once the treatment planning has been completed the treatment module 70 is used by the operator to deliver the treatment to the patient. The system provides a user interface for the operator to enter the target temperatures for the treatment of the patient. Each of the TempProbes® is therefore assigned a target temperature which is then used to determine the operation of the ablative devices.

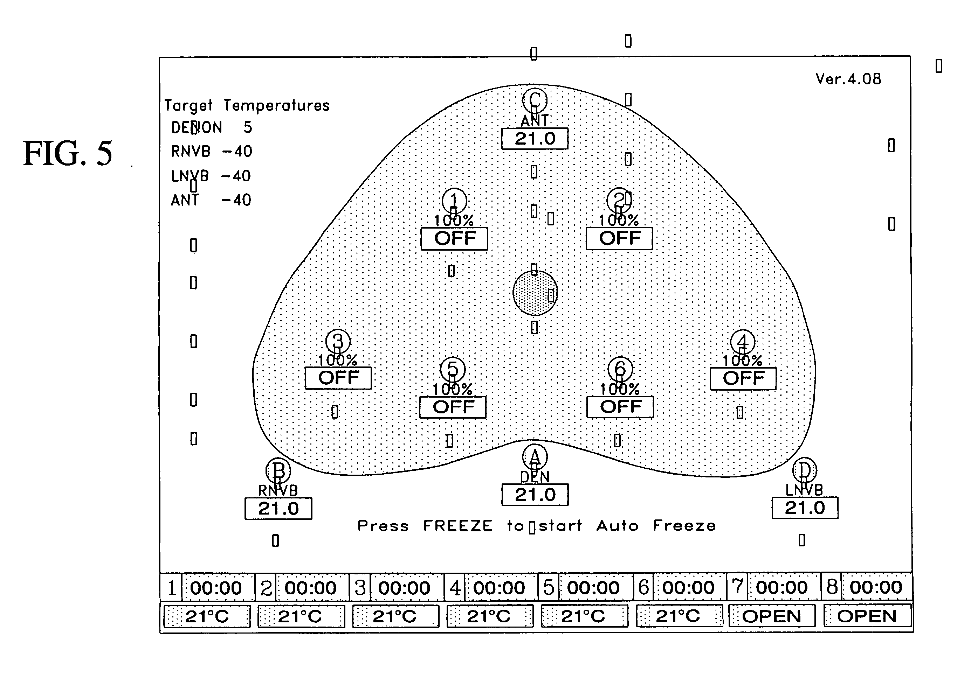

Referring to Figure 5, the cryoprobes and TempProbes® are displayed relative to the prostate and other anatomical structures of interest. The target temperatures for each of the TempProbes® are also displayed. The cryoprobes are numbered 1-6 in this figure. The TempProbes are designed A-D.

Referring again to Figure 4, the step of displaying the cryoprobes and temperature probes is denoted by block 74. The ablation cycle is started based on user input (block 76). The ablation cycle is ended, based on user input (block 78) or upon reaching target temperatures.

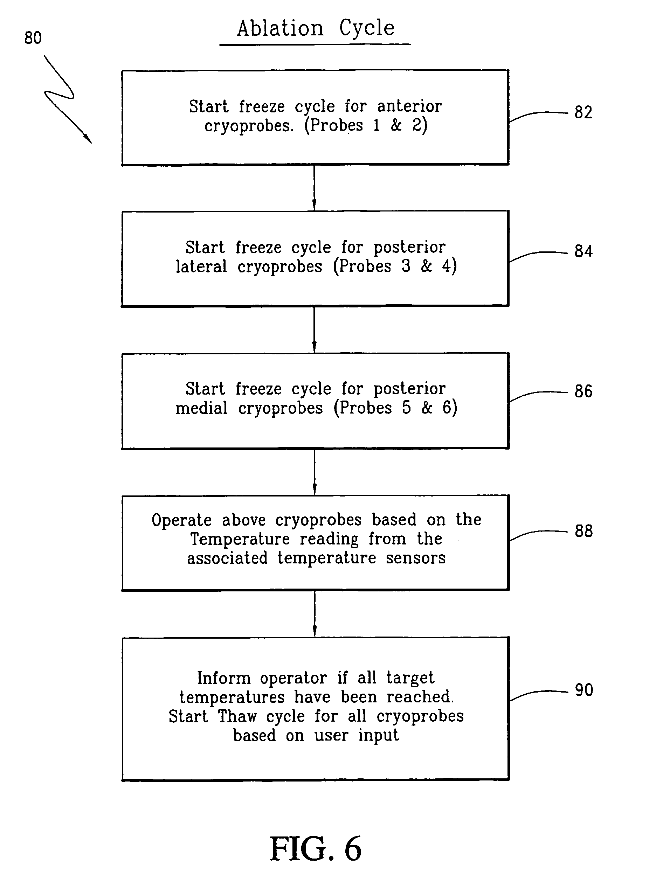

Referring now to Figure 6, a flow diagram of the ablation cycle is illustrated, designated generally as 80. A freeze cycle is started for a first anterior cryoprobe and a second anterior cryoprobe, i.e. probes 1 and 2 (block 82). A freeze cycle is started for a first posterior lateral cryoprobe and a second posterior lateral cryoprobe, i.e. probes 3 and 4 (block 84). A freeze cycle is started for a first posterior medial cryoprobe and a second posterior lateral cryoprobe, i.e. probes 5 and 6 (block 86). The cryoprobes are operated based on the temperature data from the temperature sensing devices, i.e. TempProbes (block 88). The operator is informed if all target temperatures have been reached (block 90). A thaw cycle is started for the cryoprobes based on operator input.

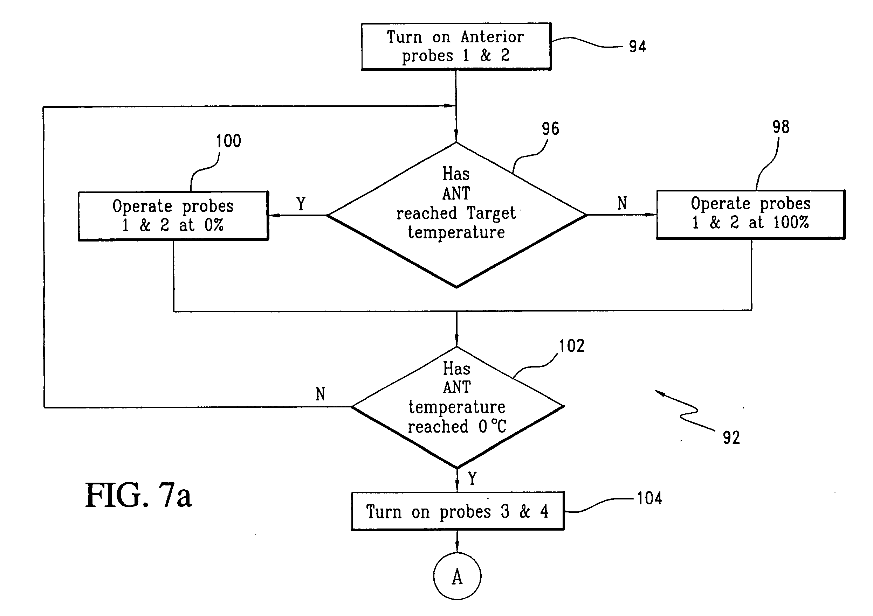

Referring now to Figure 7a, the freeze cycle for the first anterior cryoprobe and the second anterior cryoprobe is illustrated, designated generally as 92. It involves the following steps:
a) turning on the first anterior cryoprobe and the second anterior cryoprobe (block 94);
b) determining if an anterior target temperature has been reached (block 96);
c) operating the first anterior cryoprobe and the second anterior cryoprobe at a maximum rate if an anterior target temperature has not been reached (block 98);
d) operating the first anterior cryoprobe and the second anterior cryoprobe at a substantially zero rate if an anterior target temperature has been reached (block 100); and,
e) determining if the anterior target temperature has reached substantially 0°C (block 102). If yes, probes 3 and 4 are turned on (block 104).

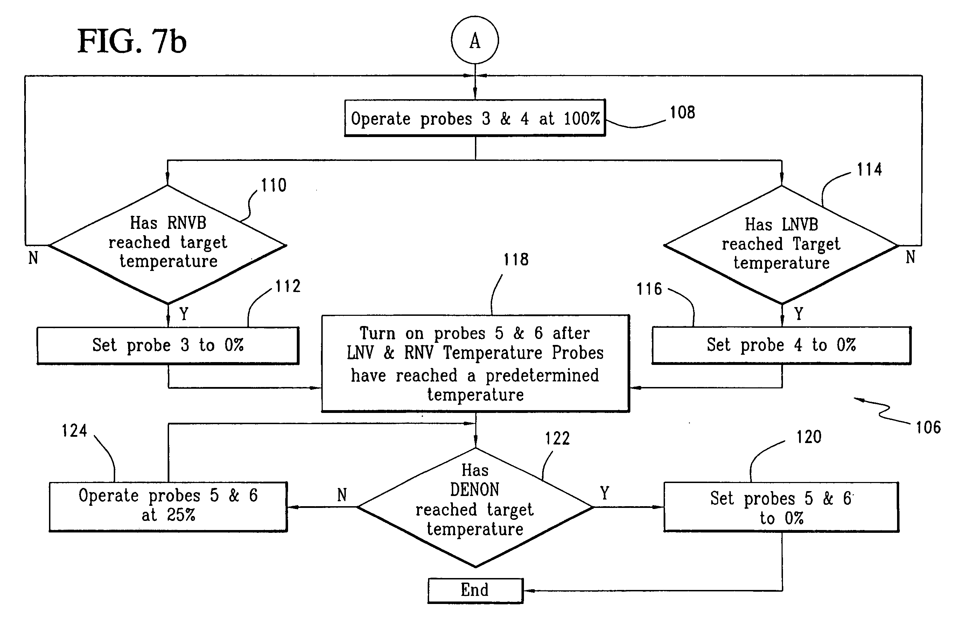

Referring now to Figure 7b, the freeze cycle for the first posterior lateral cryoprobe and the second posterior lateral cryoprobe, and for the first posterior medial cryoprobe and the second posterior lateral cryoprobe, are illustrated, designated generally as 106. These cycles involve the following steps:
a) turning on the first posterior lateral cryoprobe and the second posterior lateral cryoprobe and operating them at a maximum rate (block 108);
b) determining if a first neurovascular bundle target temperature has been reached (block 110);
c) turning off the first posterior lateral cryoprobe if the first neurovascular bundle target temperature has been reached (block 112);
d) determining if a second neurovascular bundle target temperature has been reached (block 114);
e) operating the second posterior lateral cryoprobe at a substantially zero rate if the second neurovascular bundle target temperature has been reached (block 116);
f) turning on the first posterior medial cryoprobe and the second posterior medial cryoprobe after the neurovascular TempProbes® are substantially close to their target temperatures (block 118);
g) operating the first posterior medial cryprobe and the second posterior medial cryoprobe at a power rate in a range of about 15-35%, preferably about 25% (block 124); and,
h) setting the first posterior medial cryprobe and the second posterior medial cryoprobe to a substantially zero rate (block 120) if a Denon Vieller's fascia target temperature has been reached (block 122).

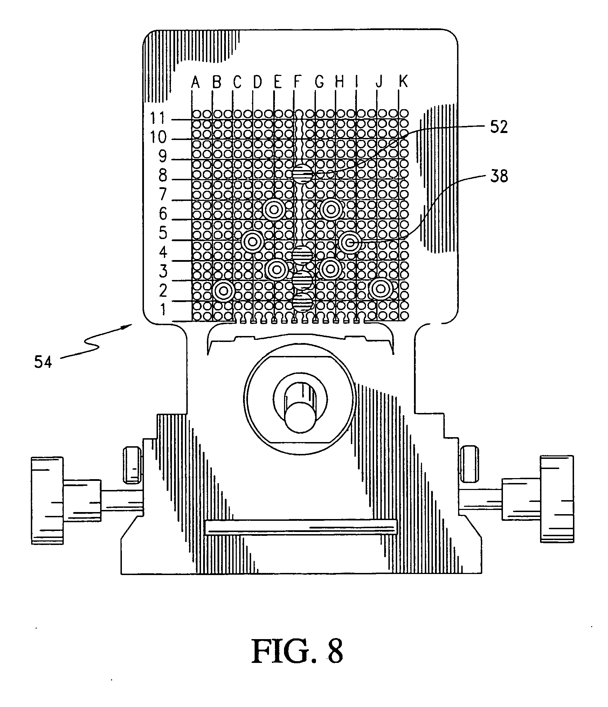

Referring now to Figure 8, an alignment assembly (also referred to as a reference plate, grid or template) is illustrated, designated generally as 54. The alignment assembly 54 utilizes an orthogonal coordinate system to position the cryoprobes and TempProbes®. Use of this alignment assembly 54 makes it possible for the cryoprobes and TempProbes® to be placed at the locations determined by the guidance module.

The cryoprobes particularly adapted for this computer guided placement are those manufactured by the present assignee, Endocare, Inc., Irvine, CA. The urethra, which passes through the prostate, is one of the anatomic structures that usually should not be frozen during this surgery. Accordingly, the urethra is protected and kept warm with the urethral warming catheter. The bladder neck sphincter and the external sphincter are also structures that should be protected from freezing, and these are protected from freezing by the warming catheter. A transrectal probe is inserted into the rectum in order to visualize the placement of the probes and the growth of the iceballs formed by the cryoprobes. (As noted above, alternative imaging means may be utilized.) To assist in placement of the cryosurgical probes, a template 21 is used which supports the probes 22 during insertion and while they are installed in the body. The patient is placed in the lithotomic position, i.e. horizontally on an operating table with legs positioned to provide access for the ultrasound probe to be inserted into the rectum and cryoprobes to be inserted through the perineal area into the prostate.

Thus, we have described a system for assisting surgeons in performing cryosurgery of the prostate by calculating optimal positions for cryoprobes and providing display based templates for overlay over an ultrasound image display, and displaying actual cryoprobe ultrasound images together with template images so that the surgeon may compare suggested and actual placement of the probes, and adjust placement accordingly. The method, which is not part of the invention, and system is described above in relation to our newly enhanced CRYOCARE™ cryosurgical system, which is provided with up to eight independently controlled argon powered cryoprobes. The enhanced CRYOCARE™ cryosurgical system utilizes the feedback described above to provide the AutoFreeze™ functionally.

The system cools the probes to cryosurgically effective temperatures (typically below - 120° C.) through Joule-Thomson cooling within the probe tips. If used for cryogenic ablation the system may be implemented with other cooling systems such as liquid nitrogen cryoprobes and mixed gas cryoprobes. The placement of probes is calculated based on this system, and the calculations may be adjusted for different systems and numbers of probes. The system may be adapted to other forms of ablation and treatment of the prostate, with adjustments in the calculations being made to account for the ablative range of the devices. Other ablative elements may include, for example, radio frequency devices, microwave devices, high intensity focused ultrasound devices, lasers, radioactive seeds and ablation agents such as chemicals, e.g. alcohol-based substances.

Although the system 10 has been described wherein the physician provides input to start and stop the ablation cycle it is understood that the treatment module may alternatively control the ablative elements automatically based upon a sensing device output signal such as, but not limited to, temperature sensing device measurements, ultrasound images of the rate of ice growth, tissue impedance measurements within the treatment zone. Such a feedback could direct the system to stop the treatment resulting in the system turning off one or more ablative elements automatically without the need for operator intervention.

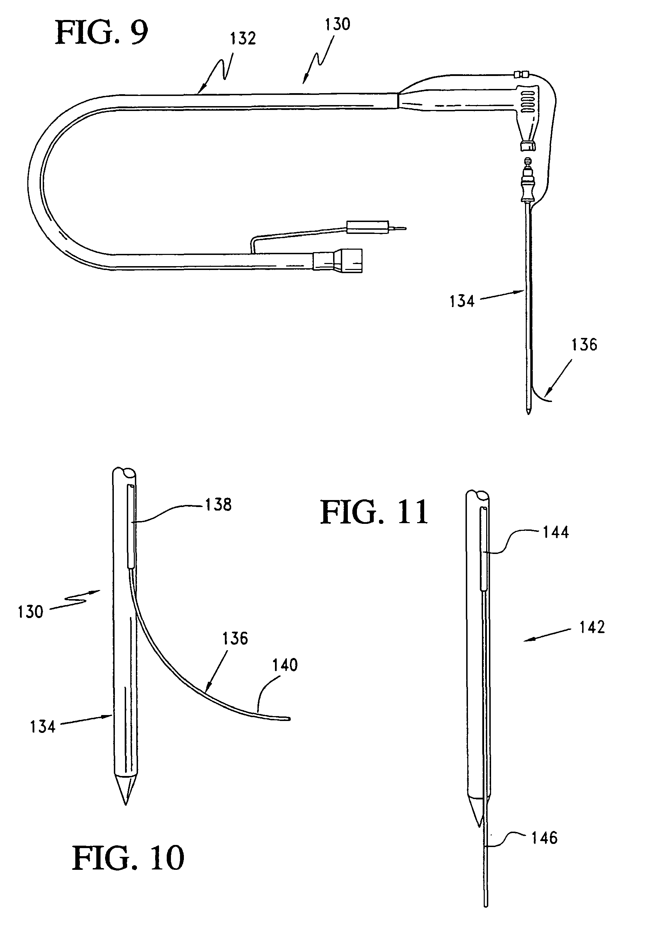

Referring now to Figure 9 an embodiment of a surgical device that comprises an integrated ablative/temperature sensing device is illustrated, designated generally as 130. The integrated ablative/temperature sensing device 130 includes a fluid supply line 132 connectable at an inlet section to a source of cryogenic fluid (not shown). A fluid connector assembly 134 is securely connected to an outlet section of the fluid supply line 132 for receiving fluid from the outlet section of the fluid supply line 132. A detachable cryosurgical probe 134 is detachably connectable to the fluid connector assembly 132. A temperature sensing device 136 is integrally attached to the ablative device, i.e. cryosurgical probe 134.

The main or longer portion of the fluid connector assembly defines a main connector assembly axis. The detachable cryosurgical probe defines a probe axis. These axes are preferably in a range of 80 degrees and 140 degrees relative to each other. Most preferably they are about 90 degrees relative to each other. Use of a right-angled system is particularly useful with computerized tomography (CT) and other image-guided (radiological) applications. Use of this cryosurgical probe system with a CT device is made easier because the detachable cryosurgical probes, fluid connector assembly, and fluid supply line can be easily contained within the confines of the CT device.

Referring now to Figure 10, an enlarged view of the distal portion of the detachable cryosurgical probe 134 is illustrated. It can be seen that the temperature sensing device 136 comprises a cannula 138 that is securely attached to the probe 134. A thermocouple 140 is positionable within the cannula 138. The thermocouple 140 is extendible from a distal portion of the cannula 138 to project outwardly from the cannula 138 at a desired distance to provide temperature profiling. The thermocouple 140 may be formed of suitable materials, as known in this field. Examples of suitable materials include constantan and copper. The thermocouple material is coupled with a shaped memory material such as Nitinol. It projects outwardly to detect temperatures at a desired radial distance from the probe 134. This data can be used to control the freeze cycle.

Referring now to Figure 11, an enlarged view of the distal portion of another embodiment of the detachable cryosurgical probe is illustrated, designated generally as 142. In this embodiment, the cannula 144 supports a thermocouple 146 that is straight. The Figure 11 embodiment is less expensive to implement.

Although Figures 9-11 illustrate a right-angled probe, use of the detachable cryosurgical probe may be with a straight probe instead. It depends on the application. Similarly, there may be an angled non-detachable system.

Thus, while the preferred embodiments of the devices have been described in reference to the environment in which they were developed, they are merely illustrative of the principles of the invention. Other embodiments and configurations may be devised without departing from the scope of the appended claims.

## Claims

1. A system (10) for providing computer guided ablation of tissue of a patient, comprising:
a. an imaging device (12) for receiving imaging data (14) from a treatment region of a patient (16), processing said imaging data and providing imaging output data (18) and imaging signals (22), said imaging output data (18) being available to an operator (20);
b. an ablative surgical computer system (24), comprising:
i) a guidance module (26) for processing said imaging signals (22) and providing a treatment guidance plan (23) to the operator (20); and,
ii) a treatment module (28) for acquiring and processing surgical device output data (30), for optimally controlling treatment parameters (32) and providing feedback information (34) to the operator (20) based on said treatment guidance plan (23);
c. a set of surgical devices (36), said set of surgical devices providing said surgical device output data (30), comprising:
i) a plurality of cryosurgical probes (38); for providing ablation of said treatment region based on said treatment parameters (32) and operator input (40); and,
ii) at least one temperature sensing device (52) for acquiring temperature data (42) from said treatment region and providing a temperature sensing device output signal, said temperature sensing device output signal being a portion of said surgical device output data (30), wherein said treatment module (28) controls the steps of:
a) acquiring target temperatures from the operator (20);
b) displaying said at least cryoprobe and temperature sensing device relative to said treatment region;
c) starting an ablation cycle (80) based on operator input (40); and,
d) ending said ablation cycle (80) based on input (40) from said operator,
and wherein said treatment guidance plan (23) is utilized for placing said plurality of cryosurgical probes (38); and said at least one temperature sensing device (52) into said treatment region, said system (10) being **characterized in that** said ablation cycle (80) comprises the steps of:
a) starting a freeze cycle for a first anterior cryoprobe and a second anterior cryoprobe (82);
b) starting a freeze cycle for a first posterior lateral cryoprobe and a second posterior lateral cryoprobe (84);
c) starting a freeze cycle for a first posterior medial cryoprobe and a second posterior medial cryoprobe (86);
d) operating said plurality of cryoprobes based on said temperature data from said at least one temperature sensing device (52); and,
e) informing said operator if all target temperatures have been reached; and,
f) starting a thaw cycle for said plurality of cryoprobes based on operator input.

2. The system of Claim 1, wherein said imaging output data (18) comprises visual imaging output data.

3. The system of Claim 1, wherein said treatment region comprises a region containing cancerous tissue.

4. The system of Claim 1, wherein said treatment region comprises a region containing tissue having an abnormal cell structure.

5. The system of Claim 1, wherein said treatment guidance plan comprises a plan that provides an optimal placement for the plurality of cryoprobes (38) and temperature sensing devices (52) relative to the treatment region.

6. The system of Claim 1, wherein said set of surgical devices (36) further comprises:
an alignment assembly associated with said plurality of cryoprobes (38) for placing said plurality of cryoprobes (38) and said at least one temperature sensing device (52) into said treatment region based on said treatment guidance plan.

7. The system of Claim 6 wherein said step of starting a freeze cycle for said first anterior cryoprobe and said second anterior cryoprobe (92), comprises the steps of:
a) turning on said first anterior cryoprobe and said second anterior cryoprobe (94);
b) determining if an anterior target temperature has been reached (96);
c) operating said first anterior cryoprobe and said second anterior cryoprobe at a maximum rate if an anterior target temperature has not been reached (100);
d) operating said first anterior cryoprobe and said second anterior cryoprobe at a substantially zero rate if an anterior target temperature has been reached (98); and,
e) determining if said anterior target temperature has reached substantially 0°C (102).

8. The system of Claim 6 wherein said steps of starting a freeze cycle for said first posterior lateral cryoprobe and said second posterior lateral cryoprobe, and for said first posterior medial cryoprobe and said second posterior medial cryoprobe (106), comprises the steps of:
a) turning on said first posterior lateral cryoprobe and said second posterior lateral cryoprobe and operating them at a maximum rate (108);
b) determining if a first neurovascular bundle target temperature has been reached (110);
c) turning off said first posterior lateral cryoprobe if said first neurovascular bundle target temperature has been reached (112);
d) determining if a second neurovascular bundle target temperature has been reached (114);
e) operating said second posterior lateral cryoprobe at a substantially zero rate if said second neurovascular bundle target temperature has been reached (116);
f) turning on said first posterior medial cryoprobe and said second posterior medial cryoprobe after neurovascular temperature readings are substantially close to their target temperatures (118);
g) operating said first posterior medial cryprobe and said second posterior medial cryoprobe at a power rate in a range of about 15-35% (124);
h) setting said first posterial medial cryoprobe and said second posterior medial cryoprobe to a substantially zero rate (120) if a Denon Vieller's fascia target temperature has been reached (122).

9. The system of Claim 6 wherein said ablation cycle (80), comprises the steps of:
a) starting a freeze cycle for a first anterior cryoprobe and a second anterior cryoprobe (82);
b) starting a freeze cycle for a first posterior lateral cryoprobe and a second posterior lateral cryoprobe (84);
c) starting a freeze cycle for a first posterior medial cryoprobe and a second posterior medial cryoprobe (86);
d) operating said plurality of cryoprobes based on said temperature data from said at least one temperature sensing device (88); and,
e) automatically controlling said freeze cycles for all of said above cryoprobes based on said target temperatures and said temperature data; and,
f) starting a thaw cycle for said plurality of cryoprobes based on user input (90).

10. The system of Claim 6 wherein said ablation cycle (80), comprises the steps of:
a) starting a freeze cycle for a first anterior cryoprobe and a second anterior cryoprobe (82);
b) starting a freeze cycle for a first posterior lateral cryoprobe and a second posterior lateral cryoprobe (84);
c) starting a freeze cycle for a first posterior medial cryoprobe and a second posterior medial cryoprobe (86);
d) operating said plurality of cryoprobes based on said temperature data from said at least one temperature sensing device (88); and,
e) automatically controlling said freeze cycles for all of said above cryoprobes based on said target temperatures and said temperature data; and,
f) starting a thaw cycle for said plurality of cryoprobes based on said temperature data (90).

11. The system of Claim 1, wherein said treatment module (28) automatically controls said plurality of cryoprobes based upon a temperature sensing device output signal.

12. The system of claim 1 further comprising:
a set of surgical devices (36), said set of surgical devices providing said surgical device output data (30) and at least one integrated ablative/temperature sensing device (52)
wherein said at least one temperature sensing device (52) is integrally attached to said a plurality of cryoprobes (38) for acquiring temperature data (42) from said treatment region and providing a temperature sensing device output signal, said temperature sensing device output signal being a portion of said surgical device output data (30),
wherein said treatment guidance plan (23) is utilized for placing said cryoprobe (38)/temperature sensing device (52) into said treatment region.

13. The system of Claim 1, wherein said at least one temperature sensing device (136) comprises a cannula (138) securely attached to said cryoprobe (134) and a thermocouple (140) positionable within said cannula (138), said thermocouple (140) being extendible from said cannula (138)at a desired distance.

14. The system of Claim 1, wherein said at least one temperature sensing device (136) comprises a cannula (138) securely attached to said cryoprobe (134) and a thermocouple (140) positionable within said cannula (138), said thermocouple (140) being extendible from a distal portion of said cannula (138) to project outwardly from said cannula (138) at a desired distance to provide temperature profiling.

## Patentansprüche

1. System (10) zum Bereitstellen einer computergeführten Abtragung von Gewebe eines Patienten, umfassend:
a. eine Bildgebungsvorrichtung (12) zum Empfangen von Bildgebungsdaten (14) von einer Behandlungsregion eines Patienten (16), Verarbeiten der Bildgebungsdaten und Bereitstellen von Bildgebungsausgabedaten (18) und Bildgebungssignalen (22), wobei die Bildgebungsausgabedaten (18) für einen Bediener (20) verfügbar sind;
b. ein ablatives chirurgisches Computersystem (24), umfassend:
i) ein Führungsmodul (26) zum Verarbeiten der Bildgebungssignale (22) und Bereitstellen eines Behandlungsanleitungsplans (23) an den Bediener (20); und
ii) ein Behandlungsmodul (28) zum Erfassen und Verarbeiten von Ausgangsdaten einer chirurgischen Vorrichtung (30) zum optimalen Steuern von Behandlungsparametern (32) und Bereitstellen von Rückmeldungsinformationen (34) an den Bediener (20) auf Grundlage des Behandlungsanleitungsplans (23);
c. einen Satz chirurgische Vorrichtungen (36), wobei der Satz chirurgische Vorrichtungen die Ausgangsdaten einer chirurgischen Vorrichtung (30) bereitstellt, umfassend:
i) eine Mehrzahl von kryochirurgischen Sonden (38) zum Bereitstellen von Abtragung der Behandlungsregion auf Grundlage der Behandlungsparameter (32) und einer Bedienereingabe (40); und
ii) wenigstens eine Temperaturmessvorrichtung (52) zum Erfassen von Temperaturdaten (42) von der Behandlungsregion und Bereitstellen eines Temperaturmessvorrichtungsausgangssignals, wobei das Temperaturmessvorrichtungsausgangssignal ein Teil der Ausgangsdaten einer chirurgischen Vorrichtung (30) ist, wobei das Behandlungsmodul (28) die folgenden Schritte steuert:
a) Erfassen von Solltemperaturen vom Bediener (20);
b) Anzeigen der wenigstens einen Kryosonde und Temperaturmessvorrichtung relativ zur Behandlungsregion;
c) Starten eines Abtragungszyklus (80) auf Grundlage einer Bedienereingabe (40); und
d) Beenden des Abtragungszyklus (80) auf Grundlage einer Eingabe (40) des Bedieners;
und wobei der Behandlungsanleitungsplan (23) dazu benutzt wird, die Mehrzahl von kryochirurgischen Sonden (38) und die wenigstens eine Temperaturmessvorrichtung (52) in der Behandlungsregion anzuordnen, wobei das System (10) **dadurch gekennzeichnet ist, dass** der Abtragungszyklus (80) folgende Schritte umfasst:
a) Starten eines Gefrierzyklus für eine erste vordere Kryosonde und eine zweite vordere Kryosonde (82);
b) Starten eines Gefrierzyklus für eine erste hintere seitliche Kryosonde und eine zweite hintere seitliche Kryosonde (84);
c) Starten eines Gefrierzyklus für eine erste hintere mittige Kryosonde und eine zweite hintere mittige Kryosonde (86);
d) Bedienen der Mehrzahl von Kryosonden auf Grundlage der Temperaturdaten von der wenigstens einen Temperaturmessvorrichtung (52); und
e) Informieren des Bedieners, wenn alle Solltemperaturen erreicht wurden; und
f) Starten eines Auftauzyklus für die Mehrzahl von Kryosonden auf Grundlage einer Bedienereingabe.

2. System nach Anspruch 1, wobei die Bildgebungsausgabedaten (16) visuelle Bildgebungsausgabedaten umfassen.

3. System nach Anspruch 1, wobei die Behandlungsregion eine Region umfasst, die Krebsgewebe enthält.

4. System nach Anspruch 1, wobei die Behandlungsregion eine Region umfasst, die Gewebe mit einer abnormen Zellstruktur enthält.

5. System nach Anspruch 1, wobei der Behandlungsanleitungsplan einen Plan umfasst, der eine optimale Anordnung der Mehrzahl von Kryosonden (38) und Temperaturmessvorrichtungen (52) relativ zur Behandlungsregion bereitstellt.

6. System nach Anspruch 1, wobei der Satz chirurgische Vorrichtungen (36) ferner Folgendes umfasst:
eine Ausrichtungsbaugruppe, die der Mehrzahl von Kryosonden (38) zugeordnet ist, um die Mehrzahl von Kryosonden (38) und die wenigstens eine Temperaturmessvorrichtung (52) auf Grundlage des Behandlungsanleitungsplans in der Behandlungsregion anzuordnen.

7. System nach Anspruch 6, wobei der Schritt des Startens eines Gefrierzyklus für die erste vordere Kryosonde und die zweite vordere Kryosonde (92) folgende Schritte umfasst:
a) Einschalten der ersten vorderen Kryosonde und der zweiten vorderen Kryosonde (94);
b) Bestimmen, ob eine vordere Zieltemperatur erreicht wurde (96);
c) Betreiben der ersten vorderen Kryosonde und der zweiten vorderen Kryosonde bei einer maximalen Rate, wenn eine vordere Solltemperatur nicht erreicht wurde (100);
d) Betreiben der ersten vorderen Kryosonde und der zweiten vorderen Kryosonde bei einer Rate von im Wesentlichen null, wenn eine vordere Solltemperatur erreicht wurde (98); und
e) Bestimmen, ob die vordere Solltemperatur im Wesentlichen 0 °C erreicht hat (102).

8. System nach Anspruch 6, wobei die Schritte des Startens eines Gefrierzyklus für die erste hintere seitliche Kryosonde und die zweite hintere seitliche Kryosonde und für die erste hintere mittige Kryosonde und die zweite hintere mittige Kryosonde (106) folgende Schritte umfassen:
a) Einschalten der ersten hinteren seitlichen Kryosonde und der zweiten hinteren seitlichen Kryosonde und Betreiben derselben bei einer maximalen Rate (108);
b) Bestimmen, ob eine erste Solltemperatur eines neurovaskulären Bündels erreicht wurde (110);
c) Ausschalten der ersten hinteren seitlichen Kryosonde, wenn die erste Solltemperatur eines neurovaskulären Bündels erreicht wurde (112);
d) Bestimmen, ob eine zweite Solltemperatur eines neurovaskulären Bündels erreicht wurde (114);
e) Betreiben der zweiten hinteren seitlichen Kryosonde bei einer Rate von im Wesentlichen null, wenn die zweite Solltemperatur eines neurovaskulären Bündels erreicht wurde (116);
f) Einschalten der ersten hinteren mittigen Kryosonde und der zweiten hinteren mittigen Kryosonde, wenn die neurovaskulären Temperaturmesswerte im Wesentlichen nahe ihrer Solltemperatur sind (118);
g) Betreiben der ersten hinteren mittigen Kryosonde und der zweiten hinteren mittigen Kryosonde bei einer Leistungsrate in einem Bereich von etwa 15 - 35 % (124);
h) Einstellen der ersten hinteren mittigen Kryosonde und der zweiten hinteren mittigen Kryosonde auf eine Rate von im wesentlichen null (120), wenn eine Solltemperatur der Denonvillier-Faszie erreicht wurde (122).

9. System nach Anspruch 6, wobei der Abtragungszyklus (80) folgende Schritte umfasst:
a) Starten eines Gefrierzyklus für eine erste vordere Kryosonde und eine zweite vordere Kryosonde (82);
b) Starten eines Gefrierzyklus für eine erste hintere seitliche Kryosonde und eine zweite hintere seitliche Kryosonde (84);
c) Starten eines Gefrierzyklus für eine erste hintere mittige Kryosonde und eine zweite hintere mittige Kryosonde (86);
d) Betreiben der Mehrzahl von Kryosonden auf Grundlage der Temperaturdaten von der wenigstens einen Temperaturmessvorrichtung (88); und
e) automatisches Steuern der Gefrierzyklen aller Kryosonden auf Grundlage der Solltemperaturen und der Temperaturdaten; und
f) Starten eines Auftauzyklus für die Mehrzahl von Kryosonden auf Grundlage einer Benutzereingabe (90).

10. System nach Anspruch 6, wobei der Abtragungszyklus (80) folgende Schritte umfasst:
a) Starten eines Gefrierzyklus für eine erste vordere Kryosonde und eine zweite vordere Kryosonde (82);
b) Starten eines Gefrierzyklus für eine erste hintere seitliche Kryosonde und eine zweite hintere seitliche Kryosonde (84);
c) Starten eines Gefrierzyklus für eine erste hintere mittige Kryosonde und eine zweite hintere mittige Kryosonde (86);
d) Betreiben der Mehrzahl von Kryosonden auf Grundlage der Temperaturdaten von der wenigstens einen Temperaturmessvorrichtung (88); und
e) automatisches Steuern der Gefrierzyklen aller Kryosonden auf Grundlage der Solltemperaturen und der Temperaturdaten; und
f) Starten eines Auftauzyklus für die Mehrzahl von Kryosonden auf Grundlage der Temperaturdaten (90).

11. System nach Anspruch 1, wobei das Behandlungsmodul (28) die Mehrzahl von Kryosonden automatisch auf Grundlage eines Temperaturmessvorrichtungsausgangssignals steuert.

12. System nach Anspruch 1, ferner umfassend:
einen Satz chirurgische Vorrichtungen (36), wobei der Satz chirurgische Vorrichtungen die Ausgabedaten einer chirurgischen Vorrichtung (30) und wenigstens einer integrierten Abtragungs-/Temperaturmessvorrichtung (52) bereitstellt.
wobei die wenigstens eine Temperaturmessvorrichtung (52) einstückig an der Mehrzahl von Kryosonden (38) angebracht ist, um Temperaturdaten (42) von der Behandlungsregion zu erfassen und ein Temperaturmessvorrichtungsausgangssignal bereitzustellen, wobei das Temperaturmessvorrichtungsausgangssignal ein Teil der Ausgabedaten einer chirurgischen Vorrichtung (30) ist,
wobei der Behandlungsanleitungsplan (23) dazu dient, die Kryosonde (38)/Temperaturmessvorrichtung (52) in der Behandlungsregion anzuordnen.

13. System nach Anspruch 1, wobei die wenigstens eine Temperaturmessvorrichtung (136) eine Kanüle (138), die sicher an der Kryosonde (134) angebracht ist, und einen Wärmefühler (140) umfasst, der innerhalb der Kanüle (138) positionierbar ist, wobei der Wärmefühler (140) über eine gewünschte Strecke aus der Kanüle (138) herausfahrbar ist.

14. System nach Anspruch 1, wobei die wenigstens eine Temperaturmessvorrichtung (136) eine Kanüle (138), die sicher an der Kryosonde (134) angebracht ist, und einen Wärmefühler (140) umfasst, der innerhalb der Kanüle (138) positionierbar ist, wobei der Wärmefühler (140) von einem distalen Teil der Kanüle herausfahrbar ist, derart, dass er um eine gewünschte Strecke aus der Kanüle (138) herausragt, um eine Temperaturprofilerstellung bereitzustellen.

## Revendications

1. Système (10) permettant une ablation guidée par ordinateur d'un tissu d'un patient, comprenant :
a. un dispositif d'imagerie (12) permettant de recevoir des données d'imagerie (14) à partir d'une région de traitement d'un patient (16), le traitement desdites données d'imagerie et la fourniture de données de sortie d'imagerie (18) et de signaux d'imagerie (22), lesdites données de sortie d'imagerie (18) étant disponibles pour un opérateur (20) ;
b. un système informatique chirurgical d'ablation (24) comprenant :
i) un module de guidage (26) servant à traiter lesdits signaux d'imagerie (22) et à procurer un plan de guidage de traitement (23) à l'opérateur (20) ; et,
ii) un module de traitement (28) permettant d'acquérir et de traiter des données de sortie de dispositif chirurgical (30), afin de contrôler de manière optimale des paramètres de traitement (32) et d'obtenir des informations en retour (34) destinées à l'opérateur (20) en fonction dudit plan de guidage de traitement (23) ;
c. un ensemble de dispositifs chirurgicaux (38), ledit ensemble de dispositifs chirurgicaux procurant lesdites données de sortie de dispositif chirurgical (30), comprenant :
i) une pluralité de sondes cryochirurgicales permettant l'ablation de ladite région de traitement en fonction desdits paramètres de traitement (32) et de l'entrée d'opérateur (40) ; et
ii) au moins un dispositif de détection de la température (52) permettant d'acquérir des données de température (42) à partir de ladite région de traitement et d'obtenir un signal de sortie du dispositif de détection de température, ledit signal de sortie de dispositif de détection de température représentant une partie desdites données de sortie du dispositif chirurgical (30), ledit module de traitement (28) contrôlant les étapes suivantes :
a) acquisition des températures cibles à partir de l'opérateur (20) ;
b) affichage de ladite au moins une cryosonde et dudit dispositif de détection de température par rapport à ladite région de traitement ;
c) démarrage d'un cycle d'ablation (80) en fonction d'une entrée d'opérateur (40) ; et
d) fin du cycle d'ablation (80) en fonction d'une entrée (40) provenant dudit opérateur ;
et où ledit plan de guidage de traitement (23) sert à placer ladite pluralité de sondes cryochirurgicales (38) et ledit au moins un dispositif de détection de la température (52) dans ladite région de traitement, ledit système (10) **se caractérisant en ce que** ledit cycle d'ablation (80) comprend les étapes suivantes :
a) démarrage d'un cycle de congélation pour une première cryosonde antérieure et pour une deuxième cryosonde antérieure (82) ;
b) démarrage d'un cycle de congélation pour une première cryosonde latérale postérieure et pour une deuxième cryosonde latérale postérieure (84) ;
c) démarrage d'un cycle de congélation pour une première cryosonde médiane postérieure et pour une deuxième cryosonde médiale postérieure (86) ;
d) activation de ladite pluralité de cryosondes en fonction de données de température provenant dudit au moins un dispositif de détection de la température (52) ; et
e) indication audit opérateur si l'ensemble des températures cibles a été atteint ; et,
f) démarrage d'un cycle de dégel pour ladite pluralité de cryosondes en fonction de l'entrée de l'opérateur.

2. Système selon la revendication 1, dans lequel lesdites données de sortie d'imagerie (18) comprennent des données de sortie d'imagerie visuelle.

3. Système selon la revendication 1, dans lequel ladite région de traitement comprend une région contenant un tissu cancéreux.

4. Système selon la revendication 1, dans lequel ladite région de traitement comprend une région contenant un tissu dont la structure cellulaire est anormale.

5. Système selon la revendication 1, dans lequel le plan de guidage de traitement contient un plan qui procure un placement optimal pour la pluralité de cryosondes (38) et des dispositifs de détection de la température (52) par rapport à la région de traitement.

6. Système selon la revendication 1, dans lequel ledit ensemble de dispositifs chirurgicaux (36) contient en outre :
un ensemble d'alignement associé à ladite pluralité de cryosondes (38) pour placer ladite pluralité de cryosondes (38) et ledit au moins un dispositif de détection de la température (52) dans ladite région de traitement en fonction dudit plan de guidage de traitement.

7. Système selon la revendication 6, dans lequel ladite étape de démarrage d'un cycle de congélation pour ladite première cryosonde antérieure et pour ladite cryosonde antérieure (92) comprend les étapes suivantes :
a) mise en marche de ladite première cryosonde antérieure et de ladite deuxième cryosonde antérieure (94) ;
b) détermination si une température cible antérieure a bien été atteinte (96) ;
c) activation de ladite première cryosonde antérieure et de ladite cryosonde antérieure à un débit maximal si aucune température cible antérieure n'a été atteinte (100) ;
d) activation de ladite première cryosonde antérieure et de ladite deuxième cryosonde antérieure à une vitesse sensiblement nulle si une température cible antérieure a été atteinte (98) ; et
e) détermination si ladite température cible antérieure a atteint sensiblement 0 °C (102).

8. Système selon la revendication 6, dans lequel lesdites étapes de démarrage d'un cycle de congélation pour ladite première cryosonde latérale postérieure et pour ladite deuxième cryosonde latérale postérieure, et pour ladite première cryosonde médiane postérieure et pour ladite deuxième cryosonde médiane postérieure (106) comprend les étapes suivantes :
a) mise en marche de ladite première cryosonde latérale postérieure et de ladite cryosonde latérale postérieure, et activationr celles-ci à une vitesse maximale (108) ;
b) détermination si une première température cible de faisceau neurovasculaire a été atteinte (110) ;
c) mise à l'arrêt de ladite première cryosonde latérale postérieure si ladite première température cible de faisceau neurovasculaire a été atteinte (112) ;
d) détermination si une deuxième température cible de faisceau neurovasculaire a été atteinte (114) ;
e) activation de ladite deuxième cryosonde latérale postérieure à une vitesse sensiblement nulle si ladite deuxième température cible de faisceau neurovasculaire a été atteinte (116) ;
f) mise en marche de ladite première cryosonde médiane postérieure et de ladite deuxième cryosonde médiane postérieure une fois que les lectures de température neurovasculaire sont sensiblement proches de leurs températures cibles (118) ;
g) activation de ladite première cryosonde médiane postérieure et de ladite deuxième cryosonde médiane postérieure à un taux de puissance compris entre 15 et 35 % (124) ;
h) réglage de ladite première cryosonde médiane et de ladite deuxième cryosonde médiane postérieure à une vitesse sensiblement nulle (120) si l'on a atteint une température cible de fascia de Denon Vieller (122).

9. Système selon la revendication 6, dans lequel ledit cycle d'ablation (80) comprend les étapes suivantes :
a) démarrage d'un cycle de congélation pour une première cryosonde antérieure et pour une deuxième cryosonde antérieure (82) ;
b) démarrage d'un cycle de congélation pour une première cryosonde latérale postérieure et pour une deuxième cryosonde latérale postérieure (84) ;
c) démarrage d'un cycle de congélation pour une première cryosonde médiane postérieure et pour une deuxième cryosonde médiane postérieure (86) ;
d) activation de ladite pluralité de cryosondes en fonction desdites données de température provenant dudit au moins un dispositif de détection de la température (88) ; et
e) contrôle automatique desdits cycles de congélation pour l'ensemble desdites cryosondes susmentionnées en fonction desdites températures cibles et desdites données de température ; et
f) démarrage d'un cycle de dégel pour ladite pluralité de cryosondes en fonction de l'entrée de l'utilisateur (90).

10. Système selon la revendication 6, dans lequel ledit cycle d'ablation (80) comprend les étapes suivantes :
a) démarrage d'un cycle de congélation pour une première cryosonde antérieure et pour une deuxième cryosonde antérieure (82) ;
b) démarrage d'un cycle de congélation pour une première cryosonde latérale postérieure et pour une deuxième cryosonde latérale postérieure (84) ;
c) démarrage d'un cycle de congélation pour une première cryosonde médiane postérieure et pour une deuxième cryosonde médiane postérieure (86) ;
d) activation de ladite pluralité de cryosondes en fonction desdites données de température provenant dudit au moins un dispositif de détection de la température (88) ; et
e) contrôle automatique desdits cycles de congélation pour l'ensemble desdites cryosondes susmentionnées en fonction desdites températures cibles et desdites données de température ; et
f) démarrage d'un cycle de dégel pour ladite pluralité de cryosondes en fonction desdites données de température (90).

11. Système selon la revendication 1, dans lequel ledit module de traitement (28) contrôle automatiquement ladite pluralité de cryosondes en fonction d'un signal de sortie de dispositif de détection de température.

12. Système selon la revendication 1, comprenant en outre :
un ensemble de dispositifs chirurgicaux (36), ledit ensemble de dispositifs chirurgicaux procurant lesdites données de sortie de dispositif chirurgical (30) et au moins un dispositif de détection de la température/ablatif intégré (52)
dans lequel ledit au moins un dispositif de détection de la température (52) est intégralement attaché à ladite pluralité de cryosondes (38) pour acquérir des données de température (42) provenant de ladite région de traitement, et pour obtenir un signal de sortie de dispositif de détection de température, ledit signal de sortie de dispositif de détection de température représentant une partie desdites données de sortie de dispositif chirurgical (30),
dans lequel ledit plan de guidage de traitement (23) sert à placer ladite cryosonde (38)/ dispositif de détection de la température (52) dans ladite région de traitement.

13. Système selon la revendication 1, dans lequel ledit au moins un dispositif de détection de la température (136) comprend une canule (138) attachée fixement à ladite cryosonde (134) et un thermocouple (140) positionnable au sein de ladite canule (138), ledit thermocouple (140) étant extensible à partir de ladite canule (138) à une distance souhaitée.

14. Système selon la revendication 1, dans lequel ledit au moins un dispositif de détection de la température (136) comprend une canule (138) attachée fixement à ladite cryosonde (134) et un thermocouple (140) positionnable au sein de ladite canule (138), ledit thermocouple (140) étant extensible à partir d'une partie distale de ladite canule (138) pour faire saillie vers l'extérieur à partir de ladite canule (138), à une distance souhaitée pour obtenir un profil de températures.
